# FASCICULE DE BREVET EUROPEEN

(11) **EP 3 105 488 B1**
(45) Date de publication et mention de la délivrance du brevet: **06.02.2019**
(21) Numéro de dépôt: 15709248.7
(22) Date de dépôt: 11.02.2015
(51) Int. Cl.: F16L 37/086

(54) **ENSEMBLE DE RACCORDEMENT FLUIDIQUE MODULAIRE.**
MODULARE FLUIDVERBINDUNGSANORDNUNG
MODULAR FLUID CONNECTION ASSEMBLY

(30) Priorité: 14.02.2014 FR 1451187
(43) Date de publication de la demande: 21.12.2016
(73) Titulaire: SARTORIUS STEDIM FMT SAS, 13400 Aubagne (FR)
(72) Inventeur: BLAKE, Florian, F-13600 La Ciotat (FR); GIBELIN, Jérémy, F-83330 Le Beausset (FR)
(74) Mandataire: Derambure Conseil
(86) Numéro de dépôt international: PCT/FR2015/050341
(87) Numéro de publication internationale: WO 2015/121589

(56) Documents cités:
- DE-U1- 9 208 976
- US-A- 5 941 577
- US-A1- 2004 189 001

## Description

L'invention concerne les raccordements fluidiques, en particulier les ensembles de raccordements fluidiques permettant de former un raccordement entre un connecteur femelle et un connecteur mâle, en vue de raccorder une conduite fluidique à une autre conduite ou à un récipient, dans le domaine des applications biopharmaceutiques.

Plus précisément, on utilise, dans le domaine biopharmaceutique, des conduites ou tubes souples, et des récipients ou contenants, qui servent à transporter et contenir des substances biopharmaceutiques diverses, avec le plus souvent des précautions d'asepsie nécessaires.
Dans le domaine des applications biopharmaceutiques, les fluides sont transportés et stockés à basse pression, c'est-à-dire généralement en dessous de 10 bars et le plus souvent en dessous de 2 bars, voire préférentiellement à une pression proche de la pression atmosphérique.

Dans le cas d'utilisation de certaines substances biopharmaceutiques, il peut être nécessaire de garantir momentanément des conditions de stérilité adéquates après que le raccordement des différents composants (conduites et/ou récipients) ait été effectué, tout en gardant la possibilité de désaccoupler le raccordement quand cela est nécessaire.

Dans d'autres cas d'utilisation de substances biopharmaceutiques, il peut être nécessaire de garantir des conditions de stérilité totale après que le raccordement des différents composants (conduites et/ou récipients) ait été effectué. À cette fin, il est commode d'utiliser des raccordements mâle-femelle indémontables après leur accouplement, ce qui permet de garantir qu'un désaccouplement non désiré ne puisse rompre les conditions de stérilité.

Par ailleurs, ce genre de raccordement doit être obtenu par un accouplement simple et rapide. En pratique, on privilégie les solutions où l'accouplement est réalisé par un mouvement de translation axiale sans rotation pour l'insertion du connecteur mâle dans le connecteur femelle.

Pour couvrir tous les cas d'utilisation possibles, on a habituellement recours à l'usage d'une part de raccordements indémontables et d'autre part de raccordements verrouillables avec possibilité de déverrouillage.

On connaît des raccordements pratiquement indémontables, par exemple à partir des documents EP-A-0566889 ou US5573279. Par ailleurs, on connaît des raccordements verrouillables avec possibilité de déverrouillage à partir du document US 5941577 et WO2010/011883.

Toutefois, les connecteurs femelle de ces différents types de raccordement sont tout à fait différents les uns des autres, notamment dans leurs formes.

Par conséquent, il est apparu un besoin de proposer des solutions modulaires permettant de pallier, au moins en partie, l'inconvénient précité de l'art antérieur connu.

Ci-après, un exposé de l'invention telle que caractérisée dans les revendications.

Selon un premier aspect, l'invention a pour objet un ensemble (autrement dit un 'kit') comprenant un connecteur femelle, pour raccordement fluide, apte et destiné à recevoir un connecteur mâle ayant un rebord annulaire extérieur de retenue, le connecteur femelle comprenant un corps avec un alésage d'axe X, et un logement formant glissière (par exemple une fente) s'étendant généralement dans un plan transversal YZ perpendiculaire à l'axe,
le logement comprenant deux rainures latérales parallèles et le logement étant débouchant à au moins une de ses extrémités selon la direction de la glissière Z,
l'ensemble (kit) comprenant un élément de verrouillage non déverrouillable, sous forme d'une bague déformable de verrouillage formée d'un seul tenant et apte à être insérée dans le logement et à verrouiller le connecteur mâle en position de couplage, sans possibilité de déverrouiller par une manoeuvre utilisateur,
l'ensemble (kit) comprenant un élément de verrouillage déverrouillable, sous forme d'un coulisseau de verrouillage apte à être inséré dans le logement et déplaçable entre une première position dite de verrouillage et une deuxième position dit de déverrouillage, le coulisseau de verrouillage comprenant un plaque de verrouillage avec un orifice central au travers duquel le connecteur maie peut passer, et un
bouton d'actuation formé intégralement avec la plaque, configuré pour être poussé vers l'axe afin de déverrouiller la connexion,
caractérisé en ce que le connecteur femelle est configuré pour accueillir indifféremment soit un élément non déverrouillable, soit un élément déverrouillable. Moyennant quoi une seule définition du connecteur femelle peut servir à former soit un raccordement déverrouillable soit un raccordement non déverrouillable. Ainsi, on forme avec seulement trois éléments à savoir le connecteur, la bague déformable et le coulisseau un ensemble modulaire permettant de former un connecteur femelle déverrouillable et un connecteur non déverrouillable pouvant recevoir un connecteur maie. Cela permet de monter dans un premier temps le connecteur femelle sur un tube souple ou sur un réservoir, poche ou contenant, puis d'insérer l'élément de verrouillage souhaité dans une étape ultérieure. Ceci permet de préparer des sous-ensembles plus en amont dans le processus, voire d'envisager des opérations de d'assemblage de ces composants par des moyens automatisés.

Selon une réalisation, le coulisseau de verrouillage et la bague déformable sont dissymétriques par rapport au plan transversal YZ, avec une forme de biseau respectivement du côté avant de la bague déformable et du côté avant du coulisseau de verrouillage. Moyennant quoi on facilite ainsi l'insertion de la portion avant du connecteur mâle à l'intérieur du connecteur femelle et de l'élément de verrouillage y inséré.

Selon une réalisation, le logement est dissymétrique par rapport au plan transversal YZ, le coulisseau de verrouillage et la bague déformable comprenant chacun au moins un ergot saillant prévu pour coopérer avec au moins une encoche correspondante ménagée dans le logement. De sorte que l'on ne peut insérer la bague déformable ou le coulisseau de verrouillage que dans un des deux sens possibles c'est-à-dire avec un détrompage face avant / face arrière. En outre, on propose ainsi une solution simple et visuelle permettant un montage adéquat, manuel ou automatisée de la bague dans le bon sens.

Selon une réalisation, le logement débouche aux deux extrémités selon Z et est symétrique par rapport au plan XY, le coulisseau de verrouillage et la bague déformable pouvant être insérés indifféremment depuis chacun des côtés du logement formant glissière. Cette disposition augmente les possibilités d'automatisation du montage.
Selon une réalisation, le coulisseau de verrouillage présente une double symétrie par rapport aux plans XZ et YZ. Ainsi, si la forme des portions de rampe du connecteur mâle le permet, on peut ainsi proposer un coulisseau qui peut être inséré dans le logement indifféremment dans les deux sens possibles avant/arrière, sans nécessité d'avoir recours à un détrompage.

Selon une réalisation, la bague déformable présente une triple symétrie, par rapport aux plans XZ, XY et YZ. De sorte qu'aucun risque d'erreur n'existe lors du montage de la bague déformable à l'intérieur du logement.

Selon une réalisation, le corps du connecteur femelle est réalisé dans une première matière plastique biocompatible, et la bague déformable est réalisée dans une seconde matière plastique, et le coulisseau de verrouillage est réalisé dans la seconde matière plastique ou dans une troisième matière plastique. De sorte que l'on peut optimiser les matériaux respectifs du connecteur femelle et de la bague de verrouillage et du coulisseau de verrouillage ; en particulier on peut choisir comme seconde matière plastique (voir troisième matière) un matériau aux performances mécaniques optimisées, concernant un compromis rigidité versus élasticité ; En outre, l'optimisation respective des deux matières permet de fabriquer ces composants avec des coûts de revient particulièrement avantageux.

Selon une réalisation, les rainures du logement comportent au moins une forme en creux configurée pour accueillir indifféremment des formes en saillie appartenant respectivement soit à la bague déformable soit au coulisseau de verrouillage. On propose ainsi un moyen très simple pour retenir l'élément de verrouillage à l'intérieur du logement.

Selon une réalisation, logement comprend des formes en creux disposées par paire symétriquement par rapport au plan XY, de manière à pouvoir accueillir la bague déformable et/ou le coulisseau quel que soit l'extrémité du logement par lequel ils sont insérés. Moyennant quoi on peut insérer la bague déformable et/ou le coulisseau indifféremment depuis chacun des côtés du logement formant glissière, avec des moyens de retenue identiques quel que soit le sens d'insertion.

Selon une réalisation, lequel la bague de verrouillage comprenant deux premières portions arquées, concaves vu de l'axe, diamétralement opposées, et deux secondes portions arquées, à courbure inversée, à savoir convexes vu de l'axe, diamétralement opposées et chacune interposée entre deux premières portions arquées. moyennant quoi après verrouillage du connecteur mâle dans le connecteur femelle, on obtient un verrouillage définitif, il n'est plus possible de déverrouiller la connexion par manipulation ou pression sur la bague de verrouillage, les secondes portions arquées n'étant pas directement accessible et les secondes portions arquées ayant tendance à renforcer le verrouillage en cas de sollicitation ou de choc.

Selon une réalisation, lequel le coulisseau de verrouillage comprenant au moins une patte flexible formée intégralement avec le coulisseau, la patte flexible rappelant le coulisseau vers la première position, et étant apte à fléchir pour laisser la plaque se déplacer vers la deuxième position, la patte flexible étant configurée pour porter de part et d'autre de l'embouchure du logement selon la direction axiale X, de sorte que l'effort de rappel est équilibré selon X et ne crée pas de couple qui pourrait avoir tendance à coincer la plaque de verrouillage.

Selon une réalisation, le connecteur femelle peut comprendre en outre dans sa portion arrière un embout tubulaire pour recevoir et maintenir un tube souple ; le connecteur femelle forme ainsi une interface modulaire pour le tube souple sur lequel il est monté.

Selon une réalisation, le connecteur femelle peut comprendre en outre dans sa portion arrière une collerette de fixation configurée pour être fixé de façon hermétique sur la paroi d'un contenant ou d'une poche; le connecteur femelle forme ainsi une interface modulaire pour le contenant ou la poche sur lequel il est monté.

On décrit maintenant brièvement les figures des dessins.
La figure 1 représente schématiquement divers composants qui peuvent être mis en oeuvre dans un système fluide biopharmaceutique.
La figure 2 montre une vue de dessus du connecteur femelle sans élément de verrouillage, conforme à l'invention.
La figure 3 est une vue en coupe axiale du dispositif de connexion de la figure 1, en position accouplée, selon la ligne de coupe II-II visible sur la figure 5.
La figure 4 est une vue en perspective d'une bague de verrouillage déformable du dispositif de connexion de la figure 3.
La figure 5 est vue en perspective d'une coupe transversale du dispositif de connexion de la figure 3.
La figure 6 montre une coupe transversale du dispositif de connexion de la figure 3.
La figure 7 est une vue en perspective d'un coulisseau de verrouillage.
La figure 8 montre une coupe transversale du dispositif de connexion avec le coulisseau de verrouillage de la figure 7.
La figure 9 est vue en perspective d'une coupe transversale du dispositif de connexion de la figure 8.
La figure 10 montre une vue de dessus du connecteur femelle sans élément de verrouillage et avec détrompage, avec deux possibilités pour la portion arrière.
La figure 11 est une vue en perspective d'une bague de verrouillage avec un détrompage.
La figure 12 est une vue en perspective d'un coulisseau de verrouillage avec détrompage.

Ci-après un exposé détaillé de plusieurs modes de réalisation de l'invention assorti d'exemples et de référence aux dessins.

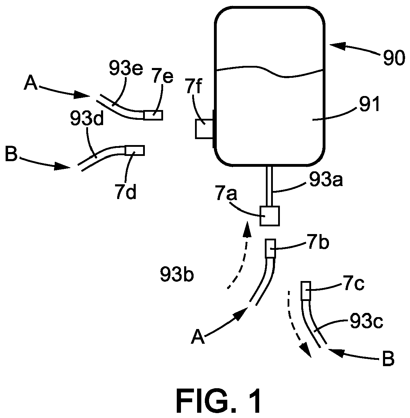

Sur la figure 1, il est représenté une poche **90** contenant une substance biopharmaceutique **91,** cette poche formant un contenant souple, semi rigide ou rigide. Dans le processus de préparation de cette poche et de son utilisation en tant que réservoir de substances biopharmaceutiques, il faut prévoir de remplir la poche avec la substance souhaitée, éventuellement transporter cette poche d'un site de préparation à un site d'utilisation, et il faut prévoir de pouvoir dispenser la substance biopharmaceutique vers l'extérieur de la poche.
Il faut remarquer que les composants mis en oeuvre dans ce genre de système biopharmaceutique peuvent être de type à usage unique, à savoir qu'une fois que le contenu de la poche a été utilisé, on jette la poche, et souvent les tubes de raccordement qui y ont été raccordés.

A cet effet, à titre d'exemple illustre actif, il peut être prévu un premier connecteur **7a,** mâle ou femelle, relié à un tube souple **93a** lui-même relié à l'espace intérieur de la poche 90. Dans ce cas, le connecteur **7a** sera muni d'un embout tubulaire apte à recevoir et retenir un tube souple **93;** par ailleurs ce premier connecteur pourra être accouplé à un second connecteur complémentaire **7b** (mâle ou femelle, de gendre opposé à 7a), de préférence avec un verrouillage non définitif, c'est-à-dire avec la possibilité de séparer les deux connecteurs une fois le remplissage de la poche effectué, couplé à un tube 93b.
De manière alternative à cette première solution, on peut disposer un second connecteur **7f** fixé directement sur la poche **90,** et dans ce cas il n'y a pas de tu plus tubes souple interposé entre le connecteur et la poche, mais on utilise une collerette de fixation **80** qui est fixée par collage ou soudure directement sur la paroi de la poche **90.**
Dans cette alternative, on peut raccorder un connecteur auxiliaire repéré **7e** monté sur un tube souple 93e sur ce second connecteur, de genre opposé, de préférence avec un verrouillage non définitif, c'est-à-dire avec la possibilité de séparer les deux connecteurs une fois le remplissage (phase 'A') de la poche effectué.

Dans la phase d'utilisation ('B'), on vient raccorder un troisième connecteur **7c** (mâle ou femelle, de gendre opposé à 7a) dans le premier connecteur 7a, pour dispenser la substance biopharmaceutique vers un site d'utilisation. Dans ce cas de figure, il peut être nécessaire de garantir des conditions de stérilité pendant la phase d'utilisation, et par conséquent on préférera avoir recours à un raccordement non démontable. Dans la variante représentée sur la gauche de la figure 1, le troisième connecteur est repéré **7d** (avec son tube 93d), et il peut former avec le second connecteur **7f** un raccordement non démontable, interdisant ainsi la déconnexion des connecteurs **7a**/7c.
Par conséquent, il est avantageux de pouvoir disposer de solutions de raccordement soit démontables soit indémontables pour satisfaire divers besoins des configurations utilisées dans le domaine des applications biopharmaceutiques.

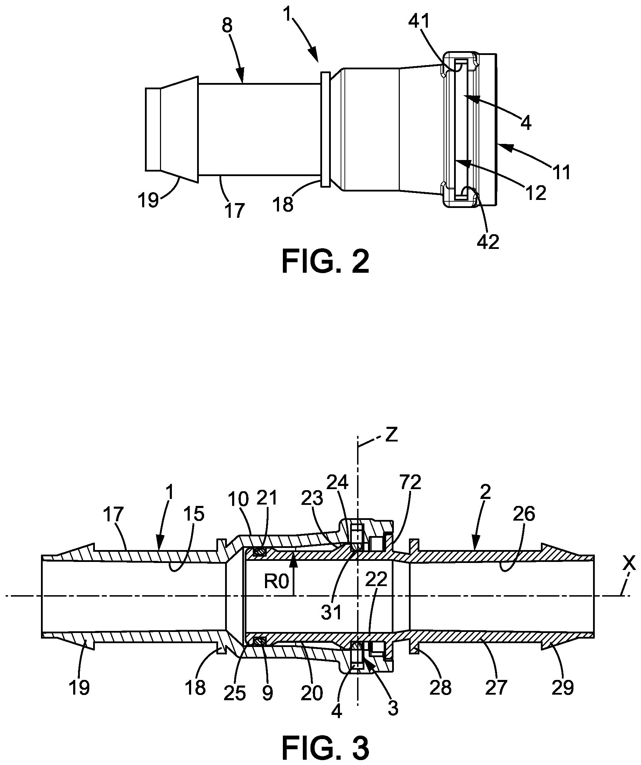

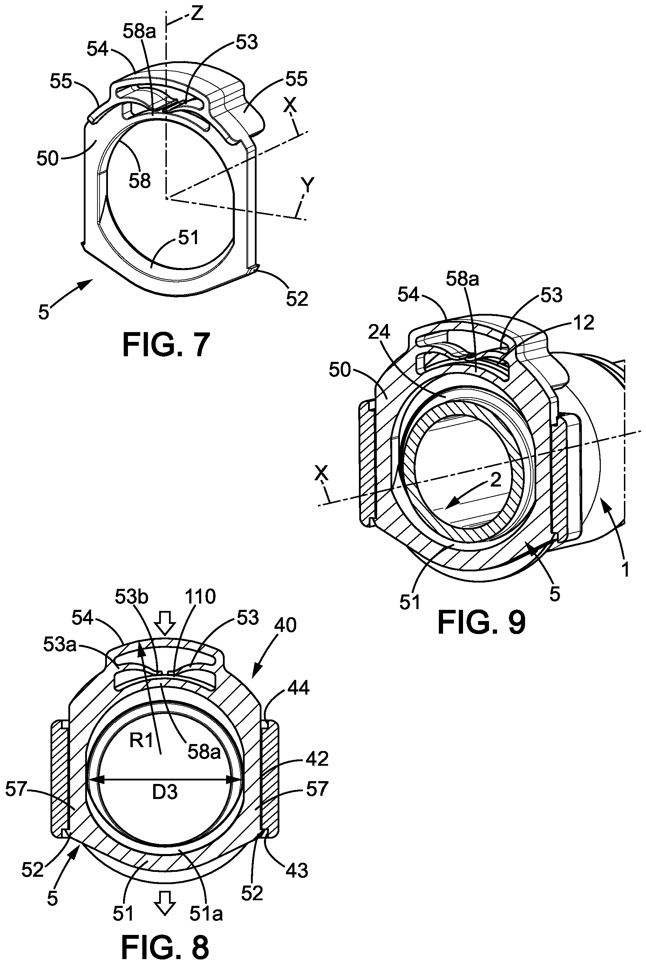

Dans les figures 2 à 12, on va maintenant décrire plus en détail les solutions proposées par la présente invention pour satisfaire les différents besoins de raccords démontables ou indémontables.

Dans l'exemple illustré, il s'agit de raccorder un connecteur femelle **1** à un connecteur mâle **2,** par un mouvement d'accouplement au cours duquel on insère le connecteur mâle dans le connecteur femelle selon une direction axiale **X.**

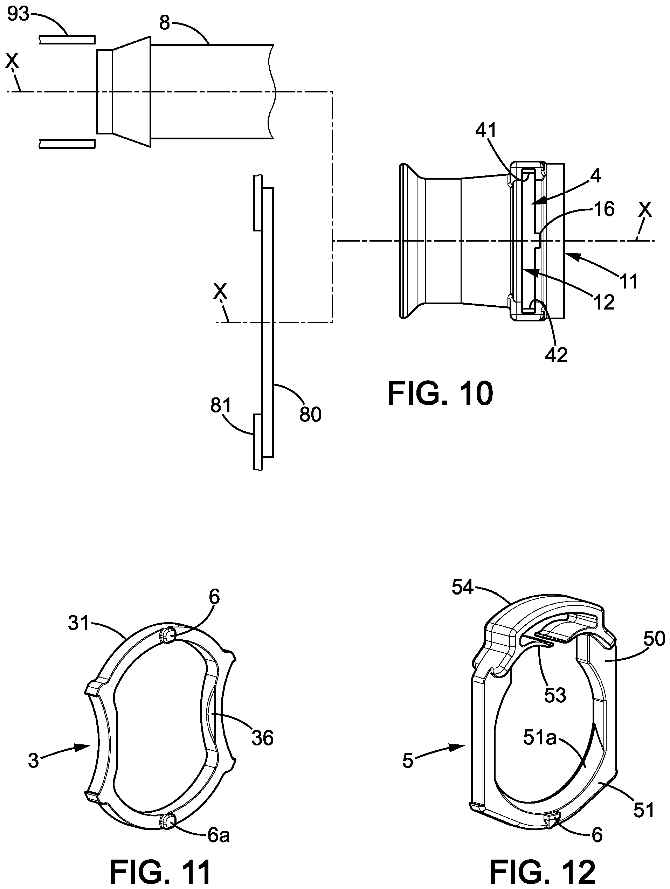

Pour chacun de ces connecteurs, dans le présent document, le terme **'avant'** désigne la partie la plus proche du connecteur opposé complémentaire, et le terme **'arrière'** désigne la partie la plus éloignée du connecteur opposé.
Dans l'exemple illustré aux figures 2 et 3, la partie arrière de chaque connecteur est formée par un embout tubulaire **8,17** destiné à recevoir un tube souple **93,** tels que ceux illustrés aux figures 1 et 10.

Le connecteur femelle 1 comprend dans sa portion arrière un embout tubulaire **17** destiné à recevoir un tube souple, et dans sa portion avant une interface de couplage avec le connecteur mâle 2, ladite interface de couplage sera décrite plus bas.
De façon similaire, le connecteur mâle **2** comprend dans sa portion arrière un embout tubulaire **27** destiné à recevoir un tube souple et dans sa portion avant une interface de couplage avec le connecteur femelle, dont il sera question plus loin.
Il faut remarquer ici que le connecteur femelle et/ou le connecteur **mâle** pourraient présenter dans leurs portions arrière, à la place d'un embout de réception de tube tel que représenté, une collerette de raccord à un récipient tel que par exemple une poche souple ou rigide ou encore un filtre.
Chacun des embouts tubulaires **17,27** est de révolution autour de l'axe X, et ils comprennent chacun un ou plusieurs crans **19,29** de rétention extérieurs pour accrocher le tube souple, et une collerette d'arrêt **18,28** sur laquelle peut venir buter le tube souple lors de son insertion sur l'embout. Comme connu en soi, on peut ajouter un collier de serrage autour du tube souple et de l'embout.

Le connecteur mâle **2** comprend un passage axial **26** de révolution autour de l'axe X, qui permet le passage du fluide transporté au travers du raccordement fluidique.
En partant de sa portion extrême avant, le connecteur mâle comprend tout d'abord un chanfrein d'entrée **25,** puis une gorge annulaire **21** destinée à recevoir un joint torique d'étanchéité **9** classique, puis une première portion intermédiaire **20** équipée de nervures longitudinales **73,** puis une partie tronconique **23** qui s'évase vers l'extérieur et vers l'arrière du connecteur mâle.
Toujours en se dirigeant vers l'arrière du connecteur, la partie tronconique **23** se prolonge par un rebord annulaire **24,** qui forme un épaulement destiné à porter sur une autre surface en contrepartie et à empêcher le retrait du connecteur mâle comme il sera vu plus loin.

En arrière du rebord annulaire anti retrait **24,** le connecteur mâle comprend une seconde portion intermédiaire **22** avec un anneau **72** équipé de quatre petits renforts **74,** ledit anneau avec ces renforts servant de moyens de préhension pour pousser le connecteur mâle en direction du connecteur femelle et servant également de butée axiale pour le mouvement d'insertion.
En arrière de l'anneau **72,** on trouve la collerette **28** et l'embout tubulaire proprement dit **27** déjà commentés.

Le connecteur femelle **1** comprend un passage axial **15** de révolution autour de l'axe X, qui permet le passage du fluide transporté au travers du raccordement fluidique.
En partant de sa portion extrême arrière, le connecteur femelle 1 comprend l'embout tubulaire **17** déjà commenté, puis un corps **10** principal destiné à recevoir la portion avant du connecteur mâle, avec un alésage de diamètre légèrement supérieur au diamètre extérieur de la portion avant de l'embout mâle **2,** de manière à pouvoir permettre l'insertion de la partie avant du connecteur mâle et de manière à pouvoir exercer une pression radiale sur le joint torique **9** pour assurer la fonction étanchéité. En vis-à-vis de la partie tronconique **23,** le corps **10** présente une dimension intérieure qui va en s'accroissant jusqu'à l'emplacement d'un logement **4** pour un élément verrouillage dont il va être question plus loin.
En effet, dans la portion avant du connecteur femelle est agencé un logement formant glissière **4** s'étendant dans un plan transversal **YZ** perpendiculaire à l'axe **X.** En l'occurrence, le logement formant glissière se présente ici comme une fente transversale. De plus, dans l'exemple illustré, cette fente débouche des deux côtés selon la direction transversale **Z** qui permet l'insertion d'une bague de verrouillage. Dans l'exemple illustré, le logement 4 comprend une première rainure **41** et une seconde de rainure **42** lesquelles se font face et son disposées symétriquement par rapport au plan **XZ.**
Enfin, le corps **10** du connecteur femelle comprend un anneau frontal 11 qui forme l'extrémité avant et sur lequel viendra buter en fin de mouvement d'insertion l'anneau **72** du connecteur mâle.

Il faut remarquer ici qu'il n'est pas strictement nécessaire que la fente 4 soit débouchante des deux côtés, en effet en variante non représentée, un des côtés pourrait être au moins partiellement fermé ce qui formerait un fond pour le logement 4 tandis que le côté opposé constituerait l'embouchure par laquelle il faut insérer les éléments de verrouillage.

Il s'agit maintenant décrire les éléments de verrouillage qui forment la différenciation fonctionnelle du raccordement, à savoir on disposera au choix d'un élément de verrouillage déverrouillable 5 et/ou d'un élément de verrouillage non déverrouillable 3.

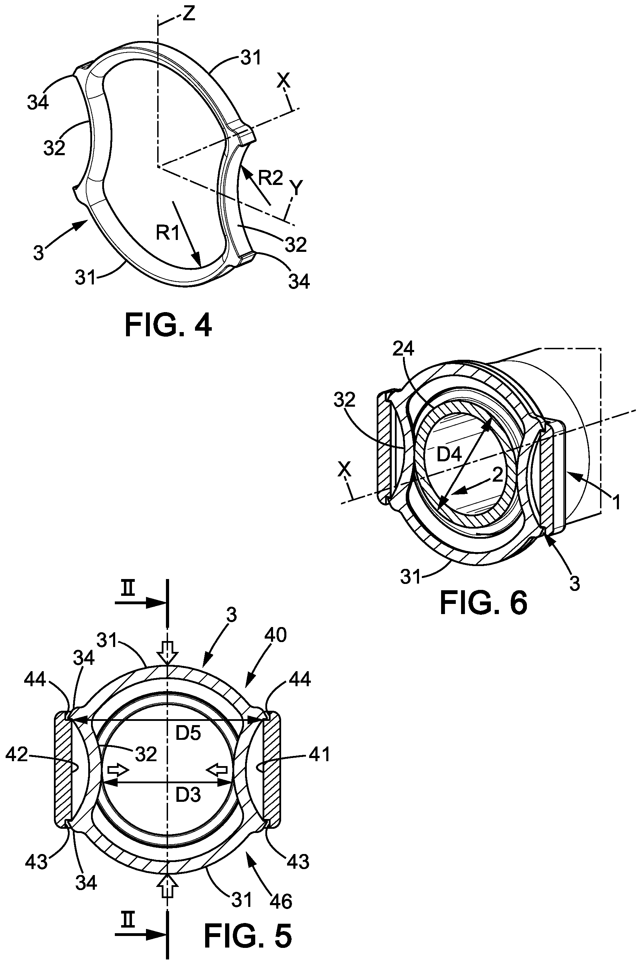

L'élément de verrouillage non déverrouillable **3** est formé par une bague de verrouillage déformable, comme illustrée aux figures 4 à 6.
La bague de verrouillage **3** déjà évoquée est destinée à être insérée dans le logement **4** susmentionné du corps du connecteur femelle. La bague de verrouillage **3** est formée comme un anneau fermé déformable, c'est-à-dire notamment qu'elle peut être déformée radialement vers l'extérieur lors du mouvement d'insertion du connecteur mâle dans le connecteur femelle.

Plus précisément, la bague de verrouillage comprend deux premières portions arquées **31,** concaves vu de l'axe **X,** diamétralement opposées, et deux secondes portions arquées **32,** à courbure inversée, à savoir convexes vu de l'axe, diamétralement opposées et chacune interposée entre deux premières portions arquées. Autrement dit, si on parcourt la bague dans la direction circonférentielle, on trouve une première portion arquée, puis une deuxième portion arquée à courbure inversée, puis une autre première portion arquée et enfin une autre deuxième portion arquée à courbure inversée.

Les premières portions arquées **31** sont concaves vu de l'axe **X,** c'est-à-dire que leur courbure est dirigée vers l'intérieur de la bague alors que secondes portions arquées **32** sont convexes vu de l'axe X c'est-à-dire que leur courbure est dirigée vers l'extérieur de la bague.

Avantageusement, les premières portions arquées **31** sont ici en forme d'arc centré sur l'axe **X,** avec un premier rayon de courbure **R1** (Fig.4) compris entre 100 % et 200 % du rayon **R0** du connecteur mâle, les secondes portions arquées **32** sont en forme d'arc avec un second rayon de courbure **R2** compris entre 70 % et 130 % du premier rayon de courbure **R1.**

Les secondes portions arquées **32** sont aptes à fléchir vers l'extérieur lors de l'insertion du connecteur mâle en particulier lorsque la forme tronconique **23** vient s'appuyer sur la bague. Après insertion complète du connecteur mâle, le rebord annulaire extérieur **24** a dépassé la bague, et la bague est rappelée vers sa position de repos et vient alors buter sur le rebord annulaire extérieur **24** de retenue pour empêcher le retrait du connecteur mâle.
Lorsque la bague de verrouillage **3** est au repos, la distance **D3** (Fig 5 et 6) entre les deux secondes portions arquées est légèrement inférieure au diamètre **D4** du connecteur mâle de la deuxième portion intermédiaire **22** en arrière du rebord annulaire **24.**

Il faut remarquer que les deuxièmes portions arquées **32** sont inaccessibles depuis l'extérieur lorsque la bague déformable est en position dans le logement **4** et seules les premières portions arquées **31** restent accessibles ; mais comme ceci est illustré à la figure 5, une pression radiale vers l'intérieur tend à accentuer la courbure des deuxièmes portions arquées et donc tend à renforcer le verrouillage ; de sorte qu'il est impossible de déverrouiller la connexion, même involontairement ou en cas de chute ou de choc du connecteur contre un objet.

Préalablement à l'insertion du connecteur mâle, la bague de verrouillage **3** peut être maintenue dans une position dite d'attente à l'intérieur du logement par différents moyens. Dans l'exemple illustré, la bague de verrouillage est maintenue dans le logement par des formes en saillie **34,** agencées substantiellement au niveau des jonctions entre les premières portions arquées et les secondes portions arquées, ce qui donne quatre formes en saillie **34,** que l'on pourrait aussi appeler crochets, becs ou griffes. Lesdites formes en saillie **34** s'étendent vers l'extérieur généralement selon la direction **Y.** Il est prévu des formes en creux **43,44** correspondantes en vis-à-vis dans les rainures **41, 42,** ces formes en creux étant destinées à recevoir les formes en saillie de la bague de verrouillage lorsque celle-ci arrive dans sa position sensiblement centrée sur l'axe **X.**
Avantageusement, on utilise les propriétés de flexibilité de la bague non seulement pour l'accouplement du connecteur mâle dans le connecteur femelle, mais aussi pour l'insertion préalable de la bague de verrouillage 3 dans la fente 4 le long de la direction Z. Plus précisément, la distance selon Y entre les extrémités des formes en saillie 34 est légèrement supérieure à la distance D5 qui sépare le fond des rainures 41, 42. Ainsi, pendant l'insertion, les formes en saillie **34** sont légèrement comprimées vers l'intérieur afin de glisser le long de l'intérieur de chacune des rainures 41,42, et de revenir vers la position de repos dès lors que les formes en saillie 34 sont en face des formes en creux **43,44** susmentionnées. Un positionnement précis de la bague n'est pas forcément requis, on peut avoir un jeu fonctionnel pour autant que la bague soit à peu près centrée sur l'axe **X.**

L'élément de verrouillage déverrouillable **5** est formé par une pièce que l'on appellera dans la suite un coulisseau de verrouillage **5.**
Le coulisseau de verrouillage **5** déjà évoqué est destiné à être inséré dans le logement **4** susmentionné du corps du connecteur femelle. Le coulisseau de verrouillage **5** est formé comme une pièce en plastique moulée comprenant une plaque de verrouillage **50,** disposée transversalement par rapport à l'axe X avec un orifice central **58** au travers duquel le connecteur mâle 2 peut passer.
De plus le coulisseau de verrouillage **5** comprend un bouton d'actuation **54** formé intégralement avec la plaque, configuré pour pouvoir être poussé vers l'axe afin de déverrouiller la connexion, par recentrage de l'orifice axial **58.**
De plus le coulisseau de verrouillage **5** comprend deux pattes flexibles **53** formées intégralement avec le coulisseau, les pattes flexibles rappelant le coulisseau vers la première position, dite position de verrouillage. Les deux pattes flexibles **53** sont aptes à fléchir pour laisser la plaque de verrouillage **50** se déplacer vers la deuxième position, dite position de déverrouillage.

Avantageusement, les deux pattes flexibles **53** sont disposées symétriquement par rapport au plan XZ. De plus, les pattes flexibles sont agencées sous le bouton d'actuation **54.** De préférence elles sont interposées entre le bouton d'activation **54** et une bordure supérieure **58a** de l'orifice principal **58.** En outre, on remarque que chacune des pattes flexibles **53** présente une courbure orientée à l'opposé du bouton-poussoir, c'est-à-dire recourbée vers le bas. De plus, chacune des pattes flexibles **53** a de préférence une section qui diminue depuis son emplanture **53a** jusqu'à son extrémité libre **53b.**
Le corps **10** présente dans la zone du logement un anneau avant 11 et un anneau arrière 12 déjà mentionnés, qui encadrent le logement. L'embouchure 40 est interposée entre un bord avant 110 de l'anneau avant 11 et un bord arrière de l'anneau arrière 12 ; la patte flexible 53 porte à la fois sur le bord avant 110 et sur le bord arrière.

Il faut remarquer ici qu'au lieu d'avoir de pattes flexibles, on pourrait tout aussi bien avoir une seule patte flexible **53** portant sur le corps du connecteur femelle de part et d'autre de l'embouchure avec les mêmes avantages.

Préalablement à l'insertion du connecteur mâle, le coulisseau de verrouillage **5** peut être maintenu dans une position d'attente à l'intérieur du logement par différents moyens. Dans l'exemple illustré, le coulisseau de verrouillage est maintenue dans le logement par des formes en saillie **52,** agencées substantiellement aux extrémités basses (opposées à la position du bouton-poussoir) des extrémités latérales de la plaque de verrouillage. Les deux formes en saillie **52,** que l'on pourrait aussi appeler crochets, becs ou griffes s'étendent vers l'extérieur généralement selon la direction **Y.** Les formes en creux 43 en vis-à-vis, situé aux extrémités des rainures **41, 42** du logement, en particulier celles référencées **43** sont destinées à recevoir les formes en saillie **52** du coulisseau lorsque celui-ci arrive dans la position l'orifice axial **58** est sensiblement centrée sur l'axe **X.**
La flexibilité du coulisseau **5** ainsi que des formes en saillie **52** permet, avant l'accouplement du connecteur mâle dans le connecteur femelle, l'insertion du coulisseau de verrouillage selon la direction **Z** depuis l'embouchure supérieure **40** du logement.
Ainsi, une fois que le coulisseau a été installé dans le logement, ces saillies 52 empêchent son extraction vers le haut, il ne peut être poussé que vers le bas au moyen du bouton d'activation 54.

Avantageusement, la portion de retenue inférieure **51** comprend une forme de rampe **51a,** autrement dit un biseau, destiné à faciliter le mouvement d'insertion du connecteur mâle qui doit déplacer le coulisseau vers le bas jusqu'à ce que le rebord annulaire de rétention **24** ait dépassé la portion de retenue **51,** ce qui provoque alors la remontée du coulisseau sous l'effet des forces de rappel des pattes flexibles **53.**

Le connecteur femelle **1** et le connecteur mâle **2** peuvent être obtenus par moulage d'une première matière plastique, en particulier des matières plastiques bio compatibles avec les diverses substances biopharmaceutiques visées, par exemple polypropylène, du polyéthylène, du polycarbonate, du polysulfone, polychlorure de vinylidène, polyethylenimine. On choisira de préférence le polyéthylène ou le polypropylène.
La bague de verrouillage déformable **3** et le coulisseau de verrouillage **5** peuvent être eux aussi réalisés dans la même matière, par exemple polybutylène téréphtalate, polypropylène, polyéthylène, polycarbonate, polyoxyméthylène ; alternativement, et avantageusement dans certains cas, on pourra choisir pour la bague déformable **3** une seconde matière plastique différente des connecteurs, typiquement du polybutylène téréphtalate ou du polycarbonate avec des propriétés mécaniques et élastiques plus ciblées, car en effet la bague de verrouillage n'est jamais en contact avec les substances transportées dans la conduite objet du raccordement.
De même, on pourra choisir pour le coulisseau **5** une seconde matière plastique différente, voire une troisième matière plastique, avec des propriétés mécaniques et élastiques plus ciblées notamment en ce qui concerne l'optimisation des pattes flexibles, car en effet le coulisseau de verrouillage n'est jamais en contact avec les substances transportées dans la conduite objet du raccordement.
On pourra optimiser, pour les pattes flexibles du coulisseau, et pour les portions arquées de la bague de verrouillage déformable, le compromis de la rigidité par rapport à l'élasticité du second matériau. On pourra aussi prendre en compte l'évolution des qualités du matériau en fonction du vieillissement naturel ou provoqué par la lumière ou d'autres rayonnements, ou d'autres agressions physico-chimiques.

Sur la figure 10, on illustre le fait que le connecteur femelle **1** et le connecteur mâle **2** (non représenté) peuvent chacun être interfacé avec un tube souple 93, ou bien directement avec la poche **90** ou le contenant au moyen de la collerette de fixation **80** qui peut être hermétiquement fixée autour d'un orifice de la paroi **81** de la poche par soudure couplage autre solution adéquate.

De plus, pour la fonction de détrompage, la paroi avant de la fente **4** est muni d'une encoche **16,** laquelle permet de rendre dissymétrique le logement vis-à-vis du plan YZ, ceci afin de procurer un détrompage de sens d'insertion pour la bague déformable et/ou le coulisseau.
De façon correspondante, la bague déformable 3 comporte au moins un ergot 6 agencé dans le milieu d'une première portion arquée 31 , faisant saillie en direction axiale X, de façon à venir s'insérer dans l'encoche 16 susmentionnée. De façon avantageuse, on pourra prévoir deux ergots 6,6A, un sur chacune des premières portions arquées sur la face avant de la bague déformable, c'est-à-dire celle où se situe la face biseautée 36.
Concernant le coulisseau 5 (Fig12), on prévoit de façon similaire un ergot 6 agencé dans le milieu de la portion de retenue 51 sur la face avant de plaque de verrouillage 50 du coulisseau, c'est-à-dire celle où se situe la face biseautée 51a.

Avantageusement dans le cadre de l'invention, on peut aussi prévoir des raccords dit « double femelle » qui comportent de chaque côté un connecteur femelle avec chacun un logement dans lequel on peut insérer soit une bague déformable soit un coulisseau.
On peut aussi utiliser le principe modulaire décrit plus haut pour le combiner avec des connecteurs aseptiques pourvus de membrane de stérilité.

On remarque que dans l'exemple illustré, le connecteur mâle peut tourner à l'intérieur du connecteur femelle autour de X, il n'y a pas de position en orientation particulière pour insérer le connecteur mâle dans le connecteur femelle.
Toutefois, il n'est pas exclu de prévoir un dispositif anti rotation qui peut par exemple être agencé entre les petits renforts de l'anneau 72 du connecteur mâle et l'anneau frontal 11 du connecteur femelle.

## Revendications

1. **Ensemble** comprenant un **connecteur femelle** (1), pour raccordement fluidique, apte et destiné à recevoir un connecteur mâle (2) ayant un rebord annulaire (24) extérieur de retenue, le connecteur femelle comprenant un **corps** (10) avec un alésage d'axe X, et un **logement** formant **glissière** (4) s'étendant généralement dans un plan transversal YZ perpendiculaire à l'axe, le logement (4) comprenant deux rainures latérales (41,42) parallèles et le logement étant débouchant à au moins une de ses extrémités (40) selon la direction de la glissière Z, l'ensemble comprenant un **élément de verrouillage déverrouillable** (5), sous forme d'un coulisseau de verrouillage (5) apte à être inséré dans le logement et déplaçable entre une première position dite de verrouillage et une deuxième position dit de déverrouillage, le coulisseau de verrouillage comprenant un plaque de verrouillage (50) avec un orifice central au travers duquel le connecteur maie peut passer, et un bouton d'actuation formé intégralement avec la plaque, configuré pour être poussé vers l'axe afin de déverrouiller la connexion,
**caractérisé en ce que** l'ensemble comprend un élément de verrouillage non déverrouillable (3), sous forme d'une bague déformable de verrouillage (3) formée d'un seul tenant et apte à être insérée dans le logement et à verrouiller le connecteur mâle en position de couplage, sans possibilité de déverrouiller par une manoeuvre utilisateur et que
le connecteur femelle est configuré pour accueillir indifféremment soit l'élément non déverrouillable, soit l'élément déverrouillable.

2. Ensemble selon la revendication 1, dans lequel le coulisseau de verrouillage (5) et la bague déformable (3) sont dissymétriques par rapport au plan transversal YZ, avec une forme de biseau respectivement du côté avant de la bague déformable et du côté avant du coulisseau de verrouillage.

3. Ensemble selon la revendication 2, dans lequel le logement (4) est dissymétrique par rapport au plan transversal YZ, le coulisseau de verrouillage (5) et la bague déformable (3) comprenant chacun au moins un ergot saillant prévu pour coopérer avec au moins une encoche correspondante ménagée dans le logement (4).

4. Ensemble selon l'une des revendications 1-3, dans lequel le logement débouche aux deux extrémités selon Z et est symétrique par rapport au plan XY, le coulisseau de verrouillage (5) et la bague déformable (3) pouvant être insérés indifféremment depuis chacun des côtés (40,46) du logement formant glissière.

5. Ensemble selon la revendication 1, dans lequel le coulisseau de verrouillage (5) présente une double symétrie par rapport aux plans XZ et YZ.

6. Ensemble selon la revendication 1 ou la revendication 5, dans lequel la bague déformable (3) présente une triple symétrie, par rapport aux plans XZ, XY et YZ.

7. Ensemble selon l'une des revendications 1-6, dans lequel le corps du connecteur femelle (1) est réalisé dans une première matière plastique biocompatible, et la bague déformable (3) est réalisée dans une seconde matière plastique, et le coulisseau de verrouillage (5) est réalisé dans la seconde matière plastique ou dans une troisième matière plastique.

8. Ensemble selon l'une des revendications 1-6, dans lequel les rainures du logement comportent au moins une forme en creux (43) configurée pour accueillir indifféremment des formes en saillie (34,52) appartenant respectivement soit à la bague déformable (3) soit au coulisseau de verrouillage (5).

9. Ensemble selon les revendication 4 et 8, dans lequel le logement (4) comprend des formes en creux (43,44) disposées par paire symétriquement par rapport au plan XY, de manière à pouvoir accueillir la bague déformable et/ou le coulisseau quel que soit l'extrémité du logement par lequel ils sont insérés.

10. Ensemble selon l'une des revendications 1-8, dans lequel la bague de verrouillage comprenant deux premières portions arquées (31), concaves vu de l'axe, diamétralement opposées, et deux secondes portions arquées (32), à courbure inversée, à savoir convexes vu de l'axe, diamétralement opposées et chacune interposée entre deux premières portions arquées, moyennant quoi après verrouillage du connecteur mâle dans le connecteur femelle, on obtient un verrouillage définitif, il n'est plus possible de déverrouiller la connexion par manipulation ou pression sur la bague de verrouillage, les secondes portions arquées n'étant pas directement accessible et les secondes portions arquées ayant tendance à renforcer le verrouillage en cas de sollicitation ou de choc.

11. Ensemble selon l'une des revendications 1-8, dans lequel le coulisseau de verrouillage comprenant au moins une patte flexible (53) formée intégralement avec le coulisseau, la patte flexible rappelant le coulisseau vers la première position, et étant apte à fléchir pour laisser la plaque se déplacer vers la deuxième position, la patte flexible (53) étant configurée pour porter de part et d'autre de l'embouchure (40) du logement selon la direction axiale X.

12. Ensemble selon l'une des revendications 1-10, comprenant en outre dans sa portion arrière un embout tubulaire (17) pour recevoir et maintenir un tube souple.

13. Ensemble selon l'une des revendications 1-10, comprenant en outre dans sa portion arrière une collerette de fixation (80) configurée pour être fixé de façon hermétique sur la paroi (81) d'un contenant ou d'une poche.

## Patentansprüche

1. Anordnung, umfassend ein weibliches Verbindungselement (1) für eine Fluidverbindung, welches dazu eingerichtet und vorgesehen ist, ein männliches Verbindungselement (2) aufzunehmen, welches einen externen ringförmigen Halterand (24) aufweist, wobei das weibliche Verbindungselement einen Körper (10) mit einer Bohrung mit Achse X sowie eine Aufnahme umfasst, welche eine Gleitführung (4) bildet, welche sich im Wesentlichen in einer transversalen Ebene YZ orthogonal zu der Achse erstreckt, wobei die Aufnahme (4) zwei parallele laterale Nuten (41, 42) umfasst und die Aufnahme an wenigstens einem ihrer Enden (40) entlang der Richtung der Gleitführung Z mündet,
wobei die Anordnung ein entriegelbares Verriegelungselement (5) in Form eines Verriegelungsschiebers (5) umfasst, welcher dazu eingerichtet ist, in die Aufnahme eingeführt zu werden und zwischen einer ersten als Verriegelungsposition bezeichneten Position und einer zweiten als Entriegelungsposition bezeichneten Position verlagerbar zu sein, wobei der Verriegelungsschieber eine Verriegelungsplatte (50) mit einer zentralen Öffnung umfasst, durch welche das männliche Verriegelungselement hindurchtreten kann, sowie einen Betätigungsknopf umfasst, welcher integral mit der Platte gebildet ist, dazu eingerichtet, in Richtung der Achse zum Entriegeln der Verbindung gedrückt zu werden,
**dadurch gekennzeichnet, dass** die Anordnung ein nicht entriegelbares Verriegelungselement (3) in Form eines deformierbaren Verriegelungsrings (3) umfasst, welcher einstückig gebildet und dazu eingerichtet ist, in die Aufnahme eingeführt zu werden und das männliche Verbindungselement in Kopplungsposition ohne Möglichkeit einer Entriegelung durch eine Betätigung eines Benutzers zu verriegeln,
und dass das weibliche Verbindungselement dazu eingerichtet ist, gleichermaßen entweder das nicht entriegelbare Element oder das entriegelbare Element zu empfangen.

2. Anordnung nach Anspruch 1, wobei der Verriegelungsschieber (5) und der deformierbare Ring (3) asymmetrisch bezüglich der transversalen Ebene YZ sind, mit einer jeweiligen Schrägflächenform der vorderen Seite des verformbaren Rings und der vorderen Seite des Verriegelungsschiebers.

3. Anordnung nach Anspruch 2, wobei die Aufnahme (4) asymmetrisch bezüglich der transversalen Ebene YZ ist, wobei der Verriegelungsschieber (5) und der deformierbare Ring (3) jeweils wenigstens einen vorstehenden Zapfen umfassen, welcher dazu vorgesehen ist, mit wenigstens einer entsprechenden Rille zusammenzuwirken, welche in der Aufnahme (4) vorgesehen ist.

4. Anordnung nach einem der Ansprüche 1-3, wobei die Aufnahme an beiden Enden entlang Z mündet und bezüglich der Ebene XY symmetrisch ist, wobei der Verriegelungsschieber (5) und der deformierbare Ring (3) gleichermaßen von jeder Seite (40, 46) der eine Gleitführung bildenden Aufnahme her eingeführt werden können.

5. Anordnung nach Anspruch 1, wobei der Verriegelungsschieber (5) eine doppelte Symmetrie bezüglich den Ebenen XZ und YZ aufweist.

6. Anordnung nach Anspruch 1 oder Anspruch 5, wobei der deformierbare Ring (3) eine dreifache Symmetrie aufweist, bezüglich den Ebenen XZ, XY und YZ.

7. Anordnung nach einem der Ansprüche 1-6, wobei der Körper des weiblichen Verbindungselements (1) aus einem ersten, biokompatiblen Kunststoffmaterial hergestellt ist und der deformierbare Ring (3) aus einem zweiten Kunststoffmaterial hergestellt ist, und der Verriegelungsschieber (5) aus dem zweiten Kunststoffmaterial oder aus einem dritten Kunststoffmaterial hergestellt ist.

8. Anordnung nach einem der Ansprüche 1-6, wobei die Nuten der Aufnahme wenigstens eine Hohlform (43) umfassen, welche dazu eingerichtet ist, gleichermaßen vorstehende Formen (34, 52) zu empfangen, welche jeweils entweder von dem deformierbaren Ring (3) oder dem Verriegelungsschieber (5) zugehörig sind.

9. Anordnung nach den Ansprüchen 4 und 8, wobei die Aufnahme (4) Hohlformen (43, 44) umfasst, welche als symmetrisches Paar bezüglich der Ebene XY in einer Weise angeordnet sind, dass sie den deformierbaren Ring und/oder den Schieber an einem beliebigen Ende der Aufnahme empfangen können, an welchem sie eingeführt werden.

10. Anordnung nach einem der Ansprüche 1-8, wobei der Verriegelungsring zwei diametral entgegengesetzte, von der Achse betrachtet konkave, erste bogenförmige Abschnitte (31) sowie zwei zweite bogenförmige Abschnitte (32) mit inverser Krümmung, das heißt von der Achse betrachtet konvex, umfasst, welche diametral entgegengesetzt und jeweils zwischen zwei ersten bogenförmigen Abschnitten eingefügt sind, wodurch nach einem Verriegeln des männlichen Verbindungselements in dem weiblichen Verbindungselement eine definitive Verriegelung erhalten wird, wobei es nicht mehr möglich ist, die Verbindung durch Betätigung oder Drücken auf den Verriegelungsring zu entriegeln, wobei die zweiten bogenförmigen Abschnitte nicht direkt zugänglich sind und die zweiten bogenförmigen Abschnitte die Tendenz haben, die Verriegelung im Fall einer Beanspruchung oder eines Stoßes zu verstärken.

11. Anordnung nach einem der Ansprüche 1-8, wobei der Verriegelungsschieber wenigstens eine flexible Klaue (53) umfasst, welche integral mit dem Schieber gebildet ist, wobei die flexible Klaue den Schieber in Richtung der ersten Position zurückführt und dazu eingerichtet ist, sich zu biegen, damit sich die Platte in Richtung der zweiten Position verlagert, wobei die flexible Klaue 53 dazu eingerichtet ist, beiderseits der Mündung 40 der Aufnahme entlang der Richtung X zu drängen.

12. Anordnung nach einem der Ansprüche 1-10, ferner umfassend in seinem hinteren Abschnitt eine rohrförmige Hülse (17) zum Aufnehmen und Halten eines flexiblen Rohrs.

13. Anordnung nach einem der Ansprüche 1-10, ferner umfassend in seinem hinteren Abschnitt eine Fixierungsschelle (80), welche dazu eingerichtet ist, in hermetischer Weise an der Wand (81) eines Behälters oder einer Tasche fixiert zu sein.

## Claims

1. Assembly comprising a female connector (1) for fluid connection, adapted for receiving a male connector (2) having a retaining external annular rim (24), the female connector comprising a body (10) with a bore of axis X, and a housing forming a slide (4) extending generally within a transverse plane YZ perpendicular to the axis, the housing (4) comprising two parallel lateral grooves (41,42) and the housing being open at least at one of its ends (40) in the direction Z of the slide,
the assembly comprising an unlockable locking element (5), in the form of a locking slider (5) adapted to be inserted into the housing and movable between a first position referred to as the locking position and a second position referred to as the unlocking position, the locking slider comprising a locking plate (50) with a central opening through which the male connector can pass, and an actuation pushbutton integrally formed with the plate, configured to be pushed toward the axis in order to unlock the connection,
**characterized in that** the assembly comprises a non-unlockable locking element (3), in the form of a deformable locking ring (3) formed as one piece and adapted to be inserted into the housing and to lock the male connector in the coupling position, with no possibility of unlocking by a user operation, and **in that** the female connector is configured to receive indiscriminately either a non-unlockable element or an unlockable element.

2. Connector according to claim 1, wherein the locking slider (5) and the deformable ring (3) are asymmetrical with respect to the transverse plane YZ, with the front side of the deformable ring and the front side of the locking slider respectively having a bevel shape.

3. Connector according to claim 2, wherein the housing (4) is asymmetrical with respect to the transverse plane YZ, the locking slider (5) and the deformable ring (3) each comprising at least one projecting pin (6) designed to engage with at least one corresponding notch in the housing (4).

4. Connector according to one of claims 1-3, wherein the housing is open at both ends along Z and is symmetrical with respect to the XY plane, so that it is possible to insert the locking slider (5) and the deformable ring (3) indiscriminately from either side (40,46) of the housing forming a slide.

5. Connector according to one of claims 1-4, wherein the locking slider (5) has a double symmetry, with respect to planes XZ and YZ.

6. Connector according to one of claims 1-5, wherein the deformable ring (3) has a triple symmetry, with respect to planes XZ, YZ, and XY.

7. Connector according to one of claims 1-6, wherein the body of the female connector (1) is made of a first biocompatible plastic material, the deformable ring (3) is made of a second plastic material, and the locking slider (5) is made of the second plastic material or of a third plastic material.

8. Connector according to one of claims 1-6, wherein the grooves of the housing comprise at least one recess (43) configured to indiscriminately accommodate projections (34, 52) that are respectively part of either the deformable ring (3) or the locking slider (5).

9. Connector according to claim 4 and claim 8, wherein the housing (4) comprises recesses (43,44) arranged in pairs, symmetrically with respect to the XY plane, so as to accommodate the deformable ring and/or the slider regardless of the end of the housing through which they are inserted.

10. Connector according to one of claims 1-8, wherein the locking ring comprises two diametrically opposed first arched portions (31), concave as viewed from the axis, and two diametrically opposed second arched portions (32) of inverse curvature, meaning convex as viewed from the axis, each of said second arched portions being interposed between two first arched portions, whereby definitive locking is obtained after the male connector is locked in the female connector: it is no longer being possible to unlock the connection by manipulation or pressure on the locking ring, as the second arched portions are not directly accessible and the second arched portions tend to reinforce the lock in case of applied force or impact.

11. Connector according to one of claims 1-8, wherein the locking slider comprises at least one flexible tab (53) integrally formed with the slider, the flexible tab returning the slider toward the first position and able to flex to allow the plate to move toward the second position, the flexible tab (53) being configured to press on each side of the mouth (40) of the housing in the axial direction X.

12. Connector according to one of claims 1-10, further comprising in its rear portion a tubular end piece (17) for receiving and retaining a flexible tube.

13. Connector according to one of claims 1-10, further comprising in its rear portion an attachment flange (80) configured to be sealingly fixed to the wall (81) of a container or pouch.
